# EUROPEAN PATENT SPECIFICATION

(11) **EP 4 159 179 B1**
(45) Date of publication and mention of the grant of the patent: **15.01.2025**
(21) Application number: 22199022.9
(22) Date of filing: 30.09.2022
(51) Int. Cl.: A61G 10/00, A61B 90/40

(54) **COLLECTIVE PROTECTION DEVICE PARTICULARLY FOR HEALTH FACILITIES SUCH AS HOSPITALS, PRIVATE CLINICS, HEALTH CENTERS AND THE LIKE**
KOLLEKTIVE SCHUTZVORRICHTUNG INSBESONDERE FÜR GESUNDHEITSEINRICHTUNGEN WIE KRANKENHÄUSER, PRIVATKLINIKEN, GESUNDHEITSZENTREN UND ÄHNLICHES
DISPOSITIF DE PROTECTION COLLECTIVE EN PARTICULIER POUR LES ÉTABLISSEMENTS DE SANTÉ TELS QUE LES HOPITAUX, CLINIQUES PRIVÉES, CENTRES DE SANTÉ ET SIMILAIRES

(30) Priority: 04.10.2021 IT 202100025310
(43) Date of publication of application: 05.04.2023
(73) Proprietor: Novamedisan Italia S.r.l., 40129 Bologna (IT)
(72) Inventor: PAZZI, Daniele, 44123 Ferrara (IT)
(74) Representative: Modiano, Micaela Nadia

(56) References cited:
- CN-A- 111 407 544
- US-A- 4 885 000
- US-A1- 2019 183 709
- US-B1- 10 842 697

## Description

The present invention relates to a collective protection device, particularly for health facilities such as hospitals, private clinics, health centers and the like. Such a device is disclosed in the US2019183709.

It is known that hospital-acquired infections are the most common and serious complications in healthcare. Hospital-acquired infections are defined as infections that arise while a patient is admitted to hospital, or discharged from hospital, and which at the time of admission were not clinically manifest or in their incubation period.

The people at risk of infection are, first and foremost, patients and, with less frequency, health facility staff, voluntary assistants, students and interns.

The principal transmission mechanisms are direct contact between a healthy person and an infected person, especially by touch; contact via droplets emitted by coughing or sneezing from an infected person to a susceptible person who is less than 50 cm away; indirect contact via a contaminated carrier (for example endoscopes or surgical instruments); transmission of the infection to multiple people simultaneously, by way of a common contaminated carrier (food, blood, infusion liquids, disinfectants etc.); by air, through microorganisms that survive in the air and are transmitted at a distance.

It is known that, in order to contain the incidence of such infections, when patients are admitted to an emergency room, for patients with symptoms that are indicative of some infections disease there is a separate area for triage, in order to prevent contact with other patients.

It is in fact essential to immediately separate such patients from the other people present in the waiting areas, and transfer them to the infectious diseases ward as soon as possible.

However, it is not rare for many hours to elapse between the time of diagnosis of the disease and the transfer to the infectious diseases ward, owing to the lack of availability of beds in the ward.

For this reason patients are therefore placed in individual rooms, if available, or the beds are separated with a curtain, ensuring that the bed of the infected patient is arranged at a distance of not less than one meter from the other beds, of uninfected patients.

It is therefore evident that this approach is based on the simple physical separation of infected patients from uninfected patients, and it does not include any measures to actually isolate the infected patient that would make it possible to limit the risk of contagion to a minimum.

The same problem is encountered in resuscitation and in intensive care wards, where generally there are approximately 10 beds to a room.

Generally, for infected patients an area is identified, which may be virtually delimited, for an adequate level of functional separation, in the knowledge that the efficacy of such containment measure is strictly linked to rigorously observing contact precautions.

It is not rare however that the healthcare personnel, usually understaffed for the number of patients present in the intensive care ward, although adequately protected by legally-mandated PPE, can find themselves having to intervene almost simultaneously on multiple patients, and may prioritize providing immediate care to the patients over taking the necessary precautions in order to prevent possible contagion between those patients.

The staff therefore does not have the time necessary to replace their PPE between one intervention on a patient and the next.

In these conditions, there is therefore a high risk of healthcare-related infections (cross-contamination), or worse again, contamination by aerosol or droplets, which can contaminate patients but also staff.

In this case too we are dealing with an area that is not isolated but only separated from the other, uncontaminated patients, who are still in the intensive care ward.

Covers are also known which enable an effective isolation of the infected patient, but such solutions, in the event of an emergency, must be removed because they do not allow direct intervention by the healthcare staff on the patient.

Removal of the cover, which effectively undoes the isolation of the patient, also brings the risk of contagion of the healthcare staff who intervene directly on the patient.

It must also be considered that hospital-acquired infections have a cost, both economic and in terms of health, both for the patient and for the health facility. The above shows the need to adopt safe healthcare practices that are capable of preventing or controlling the transmission of infections both in hospitals and in all non-hospital health facilities.

The aim of the present invention is to solve the above mentioned drawbacks, by providing a collective protection device, particularly for health facilities such as hospitals, private clinics, health centers and the like, that makes it possible to isolate potentially-infected patients from the surrounding environment.

Within this aim, an object of the invention is to provide a collective protection device, particularly for health facilities such as hospitals, private clinics, health centers and the like, that makes it possible to reduce to a minimum the risk of contagion for operators in the event of intervening directly on a potentially-infected patient.

Another object of the present invention is to provide a collective protection device, particularly for health facilities such as hospitals, private clinics, health centers and the like, that makes it possible to keep the instrumentation used to provide care for the patient, such as monitors, infusion pumps and the like, isolated from the infected patient.

Another object of the invention is to provide a collective protection device, particularly for health facilities such as hospitals, private clinics, health centers and the like, that makes it possible to prevent or control the transmission of infections between an infected patient and the surrounding environment.

Another object of the present invention is to provide a collective protection device, particularly for health facilities such as hospitals, private clinics, health centers and the like, which is of low cost, relatively simple, practically implemented and safe in use.

The invention is disclosed in the claims.

This aim and these objects are achieved by a collective protection device, particularly for health facilities such as hospitals, private clinics, health centers and the like, characterized in that it comprises:
- a base, which can be arranged above a component such as a bed, a stretcher, a cot and the like, on which a mattress for a patient can be arranged;
- a cover provided with hermetic coupling means to the perimetric edges of said base, said cover comprising closeable through openings in order to allow the entry of the hands of an operator in order to be able to intervene directly on a patient lying on the mattress;
- suction means, provided with a filtering unit, for establishing a one-way flow of the air that circulates inside the volume delimited between said base and said cover.

Further characteristics and advantages of the invention will become better apparent from the detailed description that follows of a preferred, but not exclusive, embodiment, of the collective protection device, particularly for health facilities such as hospitals, private clinics, health centers and the like, according to the invention, illustrated by way of non-limiting example in the accompanying drawings, wherein:
Figure 1 is a perspective view from above of the collective protection device, according to the invention;

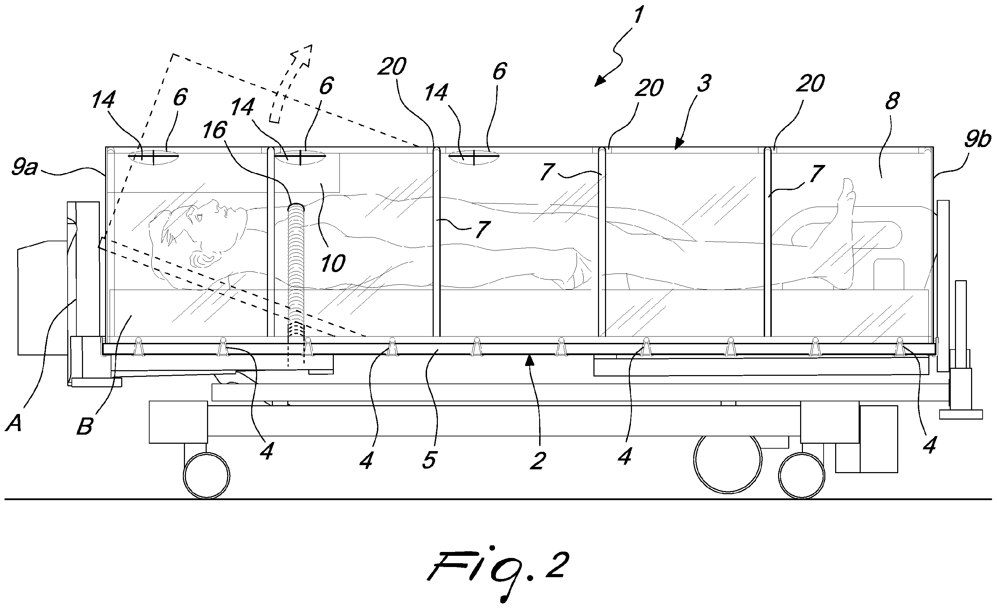

Figure 2 is a side view of the collective protection device according to the invention, under conditions of use;

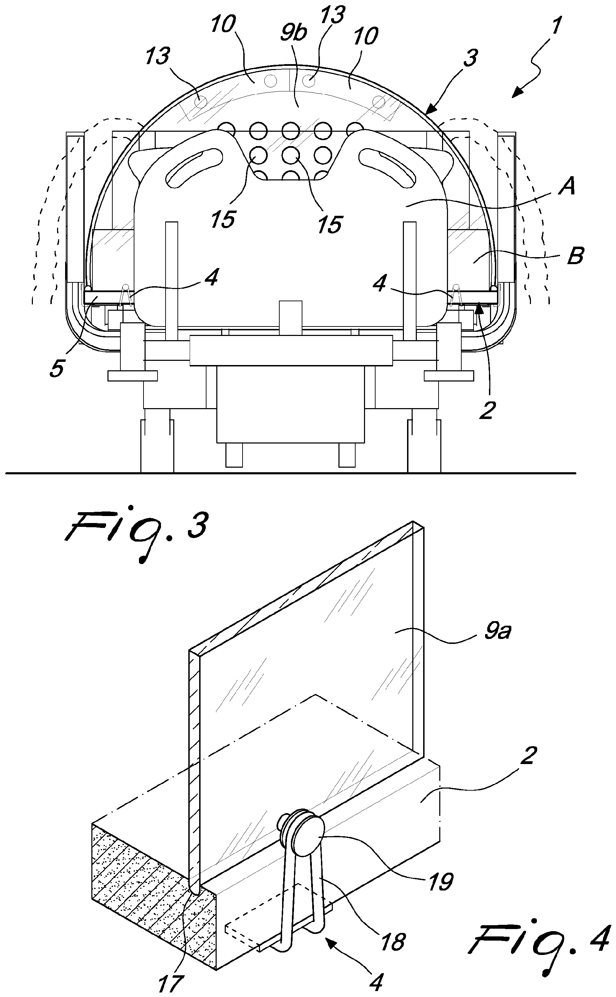

Figure 3 is a front elevation view of the collective protection device according to the invention, under conditions of use;
Figure 4 is a partially cross-sectional perspective view of the coupling means between the cover and the base of the device, according to the invention;

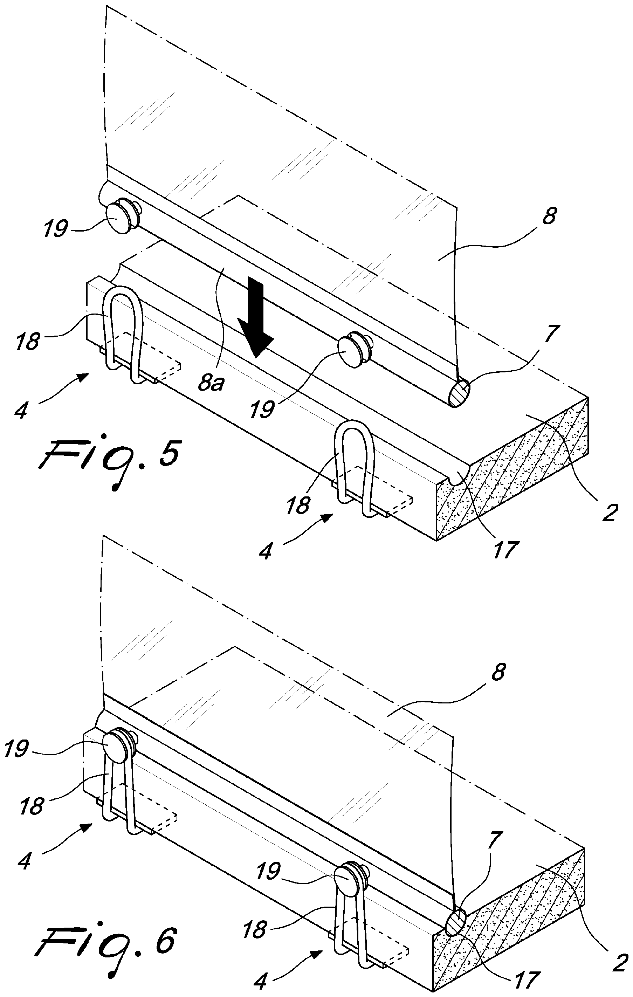

Figures 5 and 6 are partially cross-sectional perspective views of the coupling sequence between the cover and the base of the device, according to the invention.

The specific subject matter of the present discussion and the object of the protection claimed herein is a collective protection device 1, particularly for health facilities such as hospitals, private clinics, health centers and the like.

By collective protection devices (abbreviated to CPD), what is meant is devices whose function is to protect a group of persons exposed to a determined risk. Such devices are governed in Italy by Legislative Decree 81/2008 and subsequent amendments and additions.

According to the invention, the device 1 comprises a base 2, which can be arranged above a component A such as a bed, a stretcher, a cot and the like, on which a mattress B for a patient P can be arranged.

It should be noted that, in accordance with the provisions of the reference regulations for CPD, no part of a collective protection device can be in direct contact with the patient.

For this reason a mattress B is interposed between the patient P and the base 2.

The possibility is not ruled out however for the patient P to be arranged directly in contact with the base 2, if the regulations should be modified in this regard in the future.

The device 1 further comprises a cover 3 provided with hermetic coupling means 4 to the perimetric edges 5 of the base 2.

Such cover 3 comprises closeable through openings 6 in order to allow the entry of the hands of an operator in order to be able to intervene directly on the patient P.

It should be noted that the openings 6 can be four in number, arranged in pairs, respectively at the face and at the chest of the patient P, so that the operator can enter with their hands inside the cover 3 in order to perform cardiopulmonary resuscitation, and exit without generating hazardous depressions.

It has in fact been found that most diseases or infections, in particular those relative to the respiratory apparatus, generally deteriorate until a respiratory crisis occurs.

The possibility is not ruled out that the openings 6 can be greater than four in number, for example for the insertion of instrumentation, draining catheters and the like.

The device 1, according to the invention, also comprises suction means, provided with a filtering unit, for establishing a one-way flow of the air that circulates inside the volume delimited between the base 2 and the cover 3.

In this manner the air that circulates inside is conveyed in a single direction and, before exiting into the outside environment, it is suitably filtered in order to limit as far as possible the introduction of infective agents into the outside environment.

More specifically, the filtering unit can comprise filters of the HEPA (High Efficiency Particulate Arrestance) type).

The device 1 according to the invention therefore makes it possible to isolate a patient P, potentially infected, from the outside environment, while that patient is in an emergency room, awaiting transfer to a ward.

Furthermore, if while waiting the condition of the patient P should deteriorate it will be possible for the operator to intervene directly on the patient P, without removing the cover 3, thus reducing to the minimum the risk of contagion for the operator him or herself and the surrounding environment.

From a construction point of view, the cover 3 can comprise a frame 7 for supporting an enclosure 8.

Such enclosure 8 can be at least partially flexible and transparent, and comprise accommodation seats 8a for the profiled elements of the frame 7.

According to a preferred embodiment, the frame 7 can comprise two end walls 9a and 9b which are arranged respectively at the head and of the feet of the patient P.

The frame 7 will therefore prevent the enclosure 8 from falling onto the face of the patient P, by supporting it above and conveniently spaced apart from the patient P.

The possibility is furthermore not ruled out that the frame 7 can be constituted by supporting bands made of semirigid material, so that the bands, although they have a certain rigidity that makes them adapted to hold up the enclosure 8, can also be bent without being broken.

It should be noted that the enclosure 7 can be made of polyvinyl chloride.

In one embodiment of significant practical interest, the cover 3 can have, at the openings 6, an interspace 10 for the circulation of air, which is provided with closeable passages 11.

Such passages 11 can be defined in the lower wall of the interspace 10 and be substantially aligned with respective openings 6, in order to limit phenomena such as the onset of turbulence, the loss of pressure, the generation of load losses and the like.

More specifically, such interspace 10 can be subdivided by respective walls 12, arranged transversely with respect to the openings 6 and to the passages 11.

Such walls 12 can be provided with respective ports 13 for the circulation of air from the interspace 10 inward into the device 1.

Profitably, such interspace 10 can have a thickness comprised between 3 cm and 8 cm.

It should be noted that the thickness of the interspace 10, the diameter and the number of the ports 13 may vary in relation to the dimensions of the device 1, in particular to the volume delimited by the base 2 and by the cover 3.

Such device 1 can in fact be used with beds both for adults and for children; furthermore the dimensions of the device 1 can be adapted to a particular type of bed available in a hospital.

It should be noted that the opening 6 and the passages 11 can comprise deformable closure means 14 in order to limit phenomena such as the onset of turbulence, the loss of pressure, the generation of load losses and the like.

The possibility is not ruled out of having a lid to completely close the openings 6 when there is no need to intervene on the patient P.

According to an embodiment of particular effectiveness and efficiency, the suction means generate a pressure of value comprised between -10 Pa and -30 Pa.

The hermetic coupling of the cover 3 to the base 2 and the presence of the air suction means will therefore make it possible to generate a unidirectional flow of air.

The air can enter the device 1 through inlet holes 15 which are defined, on the cover 3, at the feet of the patient P, and travel up along the body of the patient up to his or her head in order to then, first through the exit holes 16 and then through the filtering unit, exit to the outside environment, free from all microbial particles.

It should be noted that the exit holes 16 can be positioned at the sides of the head of the patient P, in order to allow the operator to correctly execute resuscitation maneuvers, without being obstructed by the air suction means.

According to a preferred embodiment the exit holes 16 can be two in number, one on one side and one on the other side of the head of the patient P, in order to ensure a correct exit flow of the air.

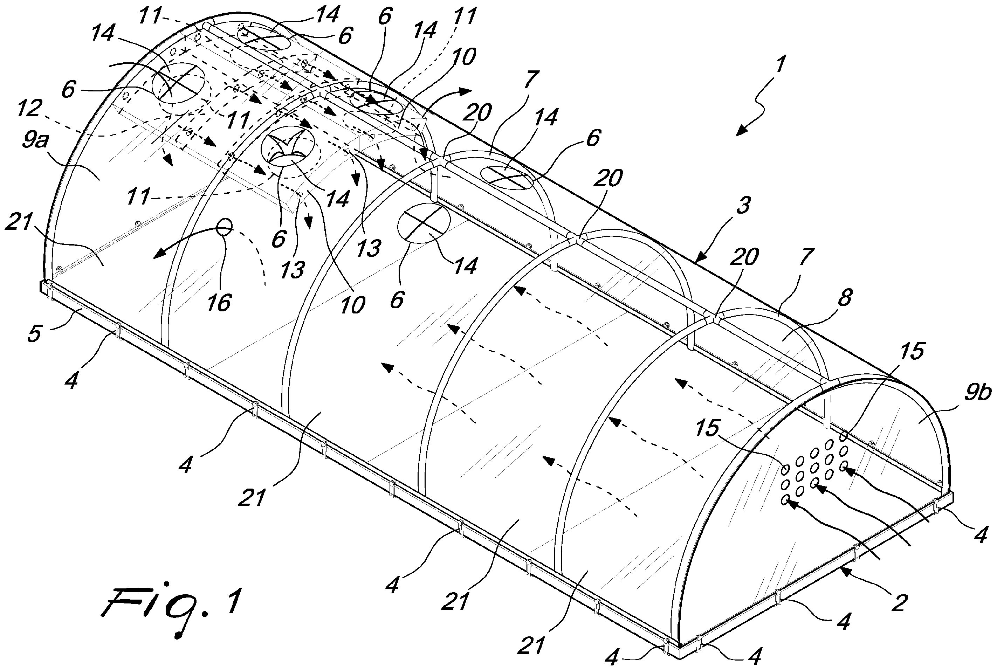

As shown in the accompanying Figure 1, the air that enters the device 1, through the openings 6, is conveyed by the action of the suction unit toward the ports 13, so that the air contained in the interspace 10 is free from infective agents.

Usefully, the coupling means 4 can be of the type preferably chosen from among a seat 17 shaped complementarily with respect to the footing of the cover 3 and provided with respective sealing gaskets, a hinge, Velcro^{®} strips, adhesive bands, annular elastomers 18 coupled detachably to respective tabs defined on the footing of the cover 3, and the like.

According to a preferred embodiment, the base 2 can have a perimetric groove 17 the shape and dimensions of which are complementary to those of the profiled element of the frame 7, and to the end walls 9a and 9b, if present; the enclosure 10 will act as a sealing gasket if it externally covers the profiled elements of the frame 7.

Conveniently, the frame 7 can comprise joining elements 20 provided with hinges with adjustable opening, in order to allow the partial lifting of the cover 3.

Furthermore, the device 1, according to the invention, can comprise sensors of the type chosen preferably from a pressure sensor, a temperature sensor, a pressure gauge and the like, in order to provide constant monitoring of the operating parameters of the device 1.

From a construction point of view, the base 2 can comprise at least two panels 21 which are mutually hinged for compacting the device 1 in the inactive configuration.

The device 1 must in fact be capable of being assembled and ready for use in a few minutes.

The presence of the hinges in the panels 21 and in the joining elements 20 can make it possible for the device 1 to follow the inclination of the underlying bed, for example when the patient needs to slightly incline the upper portion of the bed for greater comfort.

It should furthermore be noted that the suction means can be powered by an autonomous energy source, such as for example a battery.

This will make it possible to move the patient inside the device 1, from one ward to another, in a simple and rapid manner.

Advantageously, the collective protection device 1, particularly for health facilities such as hospitals, private clinics, health centers and the like, makes it possible to isolate potentially infected patients from the surrounding environment.

Effectively, the collective protection device 1, particularly for health facilities such as hospitals, private clinics, health centers and the like, makes it possible to reduce to a minimum the risk of contagion for operators in the event of intervening directly on a potentially-infected patient.

Profitably, the collective protection device 1, according to the invention, makes it possible to keep the instrumentation used to provide care for the patient, such as monitors, infusion pumps and the like, isolated from the infected patient.

Conveniently, the collective protection device 1, particularly for health facilities such as hospitals, private clinics, health centers and the like, makes it possible to prevent or control the transmission of infections between an infected patient and the surrounding environment.

Conveniently, the collective protection device 1, particularly for health facilities such as hospitals, private clinics, health centers and the like, is of low cost, relatively simple, practically implemented and safe in use.

The invention, thus conceived, is susceptible of numerous modifications and variations, all of which are within the scope of the appended claims. Moreover, all the details may be substituted by other, technically equivalent elements.

In the embodiments illustrated, individual characteristics shown in relation to specific examples may in reality be interchanged with other, different characteristics, existing in other embodiments.

In practice, the materials employed, as well as the dimensions, may be any according to requirements and to the state of the art.

## Claims

1. A collective protection device, particularly for health facilities such as hospitals, private clinics, health centers and the like, comprising:
- a base (2), which can be arranged above a component (A) such as a bed, a stretcher, a cot and the like, on which a mattress (B) for a patient (P) is configured to be arranged;
- a cover (3) provided with hermetic coupling means (4) to the perimetric edges (5) of said base (2), said cover (3) comprising closeable through openings (6) in order to allow the entry of the hands of an operator in order to be able to intervene directly on a patient (P);
- suction means, provided with a filtering unit, for establishing a one-way flow of the air that circulates inside the volume delimited between said base (2) and said cover (3),
**characterized in that** said cover (3) has, at said closeable through openings (6), an interspace (10) for the circulation of air, provided with closeable passages (11) which are substantially aligned with respective said openings (6), said interspace limiting phenomena such as the onset of turbulence, the loss of pressure, the generation of load losses and the like.

2. The device according to claim 1, **characterized in that** said cover (3) comprises a supporting frame (7) for an enclosure (8), said enclosure (8) being at least partially flexible and transparent, and comprising accommodation seats (8a) for the profiled elements of said frame (7).

3. The device according to one or more of the preceding claims, **characterized in that** said interspace (10) is divided by respective walls (12), arranged transversely to said closeable through openings (6) and said closeable passages (11), said walls (12) being provided with respective ports (13) for the circulation of the air from said interspace (10) inward into the device (1).

4. The device according to one or more of the preceding claims, **characterized in that** said closeable through opening (6) and said closeable passage (11) comprise deformable closure means (14) in order to limit phenomena such as the onset of turbulence, the loss of pressure, the generation of load losses and the like.

5. The device according to one or more of the preceding claims, **characterized in that** said air suction means generate a pressure with a value comprised between -10 Pa and -30 Pa.

6. The device according to one or more of the preceding claims, **characterized in that** said coupling means (4) are of the type preferably chosen from among a seat (17) shaped complementarily with respect to the footing of said cover (3) and provided with respective sealing gaskets, a hinge, Velcro^{®} strips, adhesive bands, annular elastomers (18) coupled detachably to respective tabs (19) defined on the footing of said cover (3), and the like.

7. The device according to claim 2, **characterized in that** said frame (7) comprises joining elements (20) provided with hinges with adjustable opening, in order to allow a partial lifting of the cover (3).

8. The device according to claim 1, **characterized in that** it comprises sensors of the type chosen preferably from a pressure sensor, a temperature sensor, a pressure gauge and the like, in order to provide constant monitoring of the operating parameters of the device (1).

9. The device according to claim 1, **characterized in that** said base (2) comprises at least two panels (21) which are mutually hinged for compacting said device (1) in the inactive configuration.

## Patentansprüche

1. Eine kollektive Schutzvorrichtung, insbesondere für Gesundheitseinrichtungen wie Krankenhäuser, Privatkliniken, Gesundheitszentren und Ähnliches, die Folgendes umfasst:
- eine Basis (2), die über einer Komponente (A) wie zum Beispiel einem Bett, einer Trage, einem Kinderbett und dergleichen angeordnet werden kann, auf der eine Matratze (B) für einen Patienten (P) ausgebildet ist, angeordnet zu werden;
- eine Abdeckung (3), ausgestattet mit hermetischen Kopplungsmitteln (4) mit den Umfangsrändern (5) der Basis (2), wobei die Abdeckung (3) verschließbare Durchgangsöffnungen (6) umfasst, um die Durchführung der Hände eines Benutzers, um direkt an einem Patienten (P) arbeiten zu können, zu ermöglichen;
- Saugmittel, ausgestattet mit einer Filtereinheit, zum Herstellen eines unidirektionalen Stroms der Luft, die in dem Volumen zirkuliert, welches zwischen der Basis (2) und der Abdeckung (3) eingegrenzt ist;
**dadurch gekennzeichnet, dass** die Abdeckung (3) an den verschließbaren Durchgangsöffnungen (6) einen Zwischenraum (10) für die Luftzirkulation hat, ausgestattet mit verschließbaren Durchlässen (11), die im Wesentlichen mit den entsprechenden Öffnungen (6) fluchten, wobei der Zwischenraum Phänomene wie das Einsetzen von Turbulenzen, den Druckverlust, die Erzeugung von Lastabwürfen und dergleichen begrenzt.

2. Die Vorrichtung gemäß Anspruch 1, **dadurch gekennzeichnet, dass** die Abdeckung (3) einen Stützrahmen (7) für eine Einfassung (8) umfasst, wobei die Einfassung (8) zumindest teilweise flexibel und transparent ist und Aufnahmesitze (8a) für die profilierten Elemente des Rahmens (7) umfasst.

3. Die Vorrichtung gemäß einem oder mehreren der obigen Ansprüche, **dadurch gekennzeichnet, dass** der Zwischenraum (10) durch entsprechende Wände (12) unterteilt ist, die quer zu den verschließbaren Durchgangsöffnungen (6) und den verschließbaren Durchlässen (11) angeordnet sind, wobei die Wände (12) zum Zwecke der Luftzirkulation von dem Zwischenraum (10) nach innen in die Vorrichtung (1) mit entsprechenden Öffnungen (13) versehen sind.

4. Die Vorrichtung gemäß einem oder mehreren der obigen Ansprüche, **dadurch gekennzeichnet, dass** die verschließbare Durchgangsöffnung (6) und der verschließbare Durchlass (11) verformbare Verschlussmittel (14) umfassen, um Phänomene wie das Einsetzen von Turbulenzen, den Druckverlust, die Erzeugung von Lastabwürfen und dergleichen zu begrenzen.

5. Die Vorrichtung gemäß einem oder mehreren der obigen Ansprüche, **dadurch gekennzeichnet, dass** die Luftsaugmittel einen Druck mit einem Wert zwischen -10 Pa und -30 Pa erzeugen.

6. Die Vorrichtung gemäß einem oder mehreren der obigen Ansprüche, **dadurch gekennzeichnet, dass** die Kopplungsmittel (4) von der Art sind, die vorzugsweise gewählt ist aus einem Sitz (17), komplementär zur Basis der Abdeckung (3) geformt und mit entsprechenden Dichtungen ausgestattet, einem Scharnier, Klettverschlüssen, Klebebändern, Elastomerringen (18), die lösbar mit entsprechenden Vorsprüngen (19) gekoppelt sind, die an der Basis der Abdeckung (3) bestimmt sind, und dergleichen.

7. Die Vorrichtung gemäß Anspruch 2, **dadurch gekennzeichnet, dass** der Rahmen (7) Verbindungselemente (20) umfasst, ausgestattet mit Scharnieren mit verstellbarer Öffnung, um ein partielles Anheben der Abdeckung (3) zu ermöglichen.

8. Die Vorrichtung gemäß Anspruch 1, **dadurch gekennzeichnet, dass** sie Sensoren von der Art umfasst, die vorzugsweise aus einem Drucksensor, einem Temperatursensor, einem Druckmessgerät und dergleichen gewählt ist, um für eine durchgehende Überwachung der Betriebsparameter der Vorrichtung (1) zu sorgen.

9. Die Vorrichtung gemäß Anspruch 1, **dadurch gekennzeichnet, dass** die Basis (2) mindestens zwei Verkleidungen (21) umfasst, die gelenkig miteinander verbunden sind, um die Vorrichtung (1) in der inaktiven Konfiguration zu verdichten.

## Revendications

1. Dispositif de protection collective, en particulier pour des établissements de santé comme des hôpitaux, des cliniques privées, des centres de santé et similaires, comprenant :
- une base (2), qui peut être agencée au-dessus d'un composant (A) comme un lit, une civière, un lit de camp et similaire, sur lequel un matelas (B) pour un patient (P) est configuré pour être agencé ;
- un recouvrement (3) pourvu de moyens de couplage hermétiques (4) aux bords périmétriques (5) de ladite base (2), ledit recouvrement (3) comprenant des ouvertures traversantes fermables (6) afin de laisser passer les mains d'un opérateur pour qu'il puisse intervenir directement sur un patient (P) ;
- des moyens d'aspiration, pourvus d'une unité de filtrage, pour établir un écoulement unidirectionnel de l'air qui circule à l'intérieur du volume délimité entre ladite base (2) et ledit recouvrement (3),
**caractérisé en ce que** ledit recouvrement (3) comporte, au niveau desdites ouvertures traversantes fermables (6), un espace intermédiaire (10) pour la circulation de l'air, pourvu de passages fermables (11) qui sont sensiblement alignés sur lesdites ouvertures (6) respectives, ledit espace intermédiaire limitant des phénomènes comme l'apparition de turbulences, la perte de pression, la génération de pertes de charge et similaires.

2. Dispositif selon la revendication 1, **caractérisé en ce que** ledit recouvrement (3) comprend un cadre de support (7) pour une enceinte (8), ladite enceinte (8) étant au moins partiellement flexible et transparente, et comprenant des sièges d'accueil (8a) pour les éléments profilés dudit cadre (7).

3. Dispositif selon une ou plusieurs des revendications précédentes, **caractérisé en ce que** ledit espace intermédiaire (10) est divisé par des parois (12) respectives, agencées transversalement auxdites ouvertures traversantes fermables (6) et auxdits passages fermables (11), lesdites parois (12) étant pourvues d'orifices (13) respectifs pour la circulation de l'air depuis ledit espace intermédiaire (10) vers l'intérieur dans le dispositif (1).

4. Dispositif selon une ou plusieurs des revendications précédentes, **caractérisé en ce que** lesdites ouvertures traversantes fermables (6) et ledit passage fermable (11) comprennent des moyens de fermeture déformables (14) afin de limiter des phénomènes comme l'apparition de turbulences, la perte de pression, la génération de pertes de charge et similaires.

5. Dispositif selon une ou plusieurs des revendications précédentes, **caractérisé en ce que** lesdits moyens d'aspiration d'air génèrent une pression d'une valeur comprise entre -10 Pa et -30 Pa.

6. Dispositif selon une ou plusieurs des revendications précédentes, **caractérisé en ce que** lesdits moyens de couplage (4) sont du type choisi préférentiellement parmi un siège (17) de forme complémentaire de l'empattement dudit recouvrement (3) et pourvu de joints d'étanchéité respectifs, une articulation, des bandes Velcro^{®}, des bandes adhésives, des élastomères annulaires (18) couplés de manière détachable à des languettes (19) respectives définies sur l'empattement dudit recouvrement (3), et des éléments similaires.

7. Dispositif selon la revendication 2, **caractérisé en ce que** ledit cadre (7) comprend des éléments de jonction (20) pourvus d'articulations à ouverture ajustable, afin de permettre un levage partiel du recouvrement (3).

8. Dispositif selon la revendication 1, **caractérisé en ce qu'**il comprend des capteurs du type choisi préférentiellement parmi un capteur de pression, un capteur de température, une jauge de pression et des éléments similaires, afin de fournir une surveillance constante des paramètres de fonctionnement du dispositif (1).

9. Dispositif selon la revendication 1, **caractérisé en ce que** ladite base (2) comprend au moins deux panneaux (21) qui sont articulés mutuellement pour compacter ledit dispositif (1) dans la configuration inactive.
